# EUROPÄISCHE PATENTSCHRIFT

(11) **EP 0 502 252 B1**
(45) Veröffentlichungstag und Bekanntmachung des Hinweises auf die Patenterteilung: **15.03.1995**
(21) Anmeldenummer: 91121044.1
(22) Anmeldetag: 09.12.1991
(51) Int. Cl.: C07C 39/17, C07C 37/20

(54) **Verfahren zur Herstellung von 9,9-Bis-(4-hydroxyphenyl-)fluoren**
Process for the preparation of 9,9-bis-(4-hydroxyphenyl)fluorene
Procédé de préparation de 9,9-bis-(4-hydroxyphenyl)fluorène

(30) Priorität: 07.03.1991 DE 4107241
(43) Veröffentlichungstag der Anmeldung: 09.09.1992
(73) Patentinhaber: RÜTGERSWERKE AKTIENGESELLSCHAFT, 60326 Frankfurt (DE)
(72) Erfinder: Orth, Winfried, Dr., W-6733 Hassloch/Pfalz (DE); Pastorek, Emmerich, Dr., W-6944 Hemsbach a. d. Bergstrasse (DE); Weiss, Wolfgang, Dr., W-6803 Neckarhausen (DE); Kleffner, Hans Werner, Dr., W-6719 Battenberg/Pfalz (DE)

(56) Entgegenhaltungen:
- EP-A- 0 180 133
- US-A- 3 546 165
- PATENT ABSTRACTS OF JAPAN, vol. 12, no. 103 (C-485)(2950), 5. April 1988; JP-A-62 230 741

## Beschreibung

Die Erfindung betrifft ein verbessertes Verfahren zur Herstellung von 9,9-Bis-(4-hydroxyphenyl-)fluoren durch Kondensation von Phenol und Fluorenon und das anschließende Verfahren zur Isolierung des Reinprodukts.

In der Kunststoffindustrie werden solche Bisphenole, insbesondere als Monomere, in großem Umfang in Polykondensationsprozessen eingesetzt. Hergestellt werden daraus beispielsweise Polyesterharze und Kunststoffe mit besonders guten hitzebeständigen Eigenschaften, die für Isolierungen elektrischer Leiter oder für hochtemperaturbeständige Überzüge verwendet werden können.

Kunststoffe mit diesen vorteilhaften Eigenschaften können jedoch nur erhalten werden, wenn zu ihrer Herstellung besonders reine Bisphenole verwendet werden.

Der wirtschaftlichen Bedeutung entsprechend wurden daher zahlreiche Versuche zur Verbesserung bekannter Synthesewege und zur Reinigung des Reaktionsprodukts unternommen.

Eine zentrale Rolle spielt in diesem Zusammenhang das von Morgan beschriebene Verfahren (P. W. Morgan, Makromolecules, 3, 536 (1970); (US 3,546,165 A1)), an das sich fast alle späteren Verfahren anlehnen. In der Kondensationsreaktion werden danach Fluorenon und Phenol im molaren Verhältnis von 1 : 4 eingesetzt, so daß Phenol während der Umsetzung gleichzeitig als Reaktand und Lösungsmittel fungiert. Die Reaktion selbst läuft in Gegenwart von β-Mercaptopropionsäure oder Mercaptoessigsäure unter Einleitung von trockenem HCl-Gas bei einer Temperatur zwischen 140 und 150 °C ab. Die hohen Temperaturen sind bei diesem Mischungsverhältnis der Reaktanden notwendig, damit die Masse rührfähig bleibt. Sie führen jedoch, wie spätere Versuche ergeben haben, zur Bildung von unerwünschten Nebenprodukten.

Die von Morgan im Anschluß an die Kondensationsreaktion vorgeschlagene Verdünnung mit Wasser oder die Wasserdampfdestillation sind zur Aufarbeitung des Reaktionsgemischs im technischen Maßstab ungeeignet. Zwar bildet sich bei der Verdünnung mit Wasser tatsächlich eine weiße Masse, jedoch ist sie kompakt, klebrig und in größeren Mengen nicht mehr zu handhaben. Auch durch eine Wasserdampfdestillation wird die gleiche klebrige Masse erhalten. Außerdem wird offensichtlich gerade durch die Behandlung mit heißem Wasser eine zusätzliche Verfärbung des Reaktionsprodukts hervorgerufen und eine Auskristallisation des Produktes verhindert.

Nach der Beschreibung von Morgan wird das abgetrennte Produkt in alkalischer Lösung aufgelöst und durch Zutropfen von Salzsäure erneut ausgefällt. Nach einer Umkristallisation aus Toluol wird 9,9-Bis-(4-hydroxyphenyl-)fluoren als weißes kristallines Produkt mit einem Schmelzpunkt von 224 bis 225 °C in einer Ausbeute von 46 bis 56 % der Theorie erhalten.

Eigene Versuche, die oben beschriebene weiße Masse in alkalischer Lösung zu lösen, gestalteten sich jedoch aufwendig und langwierig.

Ziel späterer Verfahren war es daher, einerseits die Ausbeute zu steigern, andererseits die Aufarbeitung zu vereinfachen.

Zur Ausbeutesteigerung wurden im Laufe der Zeit verschiedenste im Verfahren einsetzbare Katalysatoren ausprobiert. Dazu gehören ionisierbare Schwefelverbindungen wie z. B. Schwefelmonochlorid, Schwefelwasserstoff, verschiedenste Mercaptoverbindungen oder Alkalisulfide, die mit Säuren unter Bildung von Schwefelwasserstoff reagieren. Eingesetzt wurden auch Friedel-Crafts-Katalysatoren wie z. B. ZnCl₂, CaCl₂, AlCl₃ oder SnCl₄, die in Gegenwart von HCl wirksam sind.

Es stellte sich jedoch heraus, daß nicht nur die Wahl des Katalysators die Ausbeutemenge und die Produktqualität entscheidend beeinflussen, sondern insbesondere die Reaktionstemperatur und das molare Verhältnis der Reaktanden zueinander. Hohe Ausbeuten konnten daher sowohl mit Friedel-Crafts-Katalysatoren als auch mit der durch Morgan vorbeschriebenen Mercaptopropionsäure erzielt werden, wenn die Reaktionstemperatur unter 100 °C, insbesondere zwischen 30 und 90 °C gehalten und Phenol in der vier- bis achtfachen molaren Menge bezogen auf das Fluorenon eingesetzt wurde.

In diesem Temperaturbereich wird auch die Kondensationsreaktion in dem in der Offenlegungsschrift DE-OS 34 39 484 beschriebenen Verfahren durchgeführt. Fluorenon und Phenol werden dabei im molaren Verhältnis von 1 : 4 bis 1 : 6 in Gegenwart von β-Mercaptopropionsäure und konzentrierter Schwefelsäure umgesetzt. Durch das sich anschließende Aufarbeitungsverfahren wird nach den Angaben der Anmelderin eine Rohausbeute von etwa 97 bis 98 % erhalten. Darin ist jedoch noch ein hoher Anteil unerwünschter Nebenprodukte enthalten, die durch Dimerisierung und Substitution entstanden sind. Die weitere Aufarbeitung erfolgt, indem nach beendeter Kondensationsreaktion Methanol hinzugefügt und erst anschließend in kaltes Wasser gegossen wird. Auch hier scheidet sich die ölige Masse ab. Die überstehende wäßrige Methanol-Schwefelsäure-Phenol-Lösung wird abgetrennt und der Rückstand noch zweimal mit Wasser gewaschen und mit Ammoniumcarbonatlösung neutralisiert.

Noch anhaftendes Phenol wird durch mehrmaliges Aufkochen mit Wasser entfernt. Nach dem Trocknen und nochmaliger Umkristallisation mit Isopropylalkohol wird ein Produkt mit einem Schmelzpunkt von 223 °C erhalten.

Dieses Verfahren besitzt jedoch gravierende Nachteile. Bei der Aufarbeitung des Reaktionsprodukts fallen sehr große Mengen mit Phenol und Schwefelsäure verunreinigten Wassers an, die aufgrund der Gewässerschutzverordnungen einer besonderen Aufarbeitung zugeführt werden müssen. Außerdem ist auch bei diesem Verfahren das nach der Wasserzugabe anfallende, verklumpte Rohprodukt schwer zu handhaben. Es läßt sich nur schwer abfiltrieren und zur weiteren Reinigung waschen und neutralisieren.

Aufgabe der Erfindung ist es daher, ein preiswertes, leicht durchführbares Verfahren zur Herstellung von 9,9-Bis-(4-hydroxyphenyl-)fluoren in hoher Ausbeute und Reinheit zur Verfügung zu stellen, daß es erlaubt, zur Produktreinigung verwendete Lösungsmittel in einfacher Weise abzutrennen, so daß sie möglichst erneut im Verfahren eingesetzt werden können.

Die Lösung der Aufgabe erfolgt durch ein Verfahren gemäß Anspruch 1 oder 5 und seiner Ausgestaltung gemäß den Ansprüche 2 bis 4.

Es wurde gefunden, daß die im Reaktionsgemisch enthaltene Salzsäure gemeinsam mit dem entstandenen Reaktionswasser leicht unter vermindertem Druck abdestilliert werden kann, wenn nach der von Morgan beschriebenen Methode gearbeitet wird. Dieses hat den Vorteil, daß unter wesentlich milderen Bedingungen gearbeitet werden kann, und sich weniger unerwünschte Nebenprodukte durch Dimerisierung und Substitution bilden, als wenn mit konzentrierter Schwefelsäure gearbeitet wird.

Gleichzeitig können dadurch Nachteile vermieden werden, die bei der Produktaufarbeitung und durch die dabei anfallenden Lösungsmittelmengen auftreten, wenn in Gegenwart von Schwefelsäure oder von Friedel-Crafts-Katalysatoren gearbeitet wird.

Wie sich gezeigt hat, läuft die Kondensationsreaktion von Fluoren und Phenol in Gegenwart von HCl bereits bei 30 °C in ausreichender Geschwindigkeit ab. Bevorzugt wird bei Temperaturen zwischen 50 und 60 °C gearbeitet, da in diesem Bereich die Bildung von Nebenprodukten niedrig ist, und die Reaktionszeit bis zum vollständigen Umsatz von Fluorenon annehmbar kurz ist.

Werden die Reaktanden Fluorenon und Phenol, wie bei Morgan angegeben, im molaren Verhältnis von 1 : 4 zur Reaktion eingesetzt, verfestigt sich gegen Ende die Reaktionsmasse. Wird dagegen Phenol in der sechs- bis achtfachen molaren Menge, bezogen auf Fluorenon, eingesetzt, bleibt die Masse durchgehend rührfähig.

Nach der Reaktion läßt sich zwar das Reaktionswasser gemeinsam mit gelöster Salzsäure unter vermindertem Druck abdestillieren, aber für das im Überschuß eingesetzte Phenol ist das jedoch nicht zu empfehlen. Wird es völlig abdestilliert, erhält man eine kompakte Masse als Destillationsrückstand, die nicht mehr weiter zu verarbeiten ist.

Überraschenderweise wurde nun gefunden, daß dieses durch Zusatz einer geringen Menge eines Polyalkylenglykols vermieden werden kann. Der Destillationsrückstand bleibt dünnflüssig und rührbar und wird aus einem mit diesem Glykol mischbaren Lösungsmittel, wie z. B. Dichlorethan, Toluol, Ether oder Nitromethan in an sich bekannter Weise auskristallisiert.

Durch das erfindungsgemäße Verfahren ist es möglich, zur Reaktion im Überschuß eingesetztes Phenol aus dem Reaktionsgemisch abzudestillieren und es in den Prozeß wieder zurückzuführen.

Da vor dem Abdestillieren des Phenols die im Reaktionsgemisch enthaltene Salzsäure gemeinsam mit dem Reaktionswasser entfernt werden kann, entfällt die sonst übliche Wasserwäsche ebenso wie die Wasserdampfdestillation.

Als weiterer Vorteil erweist sich, daß die Auskristallisation mit wesentlich geringeren Lösungsmittelmengen durchgeführt werden kann, als wenn die Reaktionsmasse nach einer Wasser- oder Methanol-Wasserbehandlung bzw. nach dem vollständigen Abdestillieren des Phenols aus einem entsprechenden Lösungsmittel auskristallisiert wird. Wird z. B. aus Toluol auskristallisiert, benötigt man im erfindungsgemäßen Verfahren nur noch 1/6 der sonst üblichen Menge.

Als Hilfsmittel zur Abtrennung des Reaktionsprodukts können Polyethylenglykole der Formel H(-O-CH₂-CH₂-)ₙ OH mit n = 2 bis 13, also solche mit glycerinähnlichem Fließverhalten, eingesetzt werden. Eingesetzt werden können aber auch entsprechende Polypropylenglykole oder andere Polyalkylenglykole.

Besonders bevorzugt wird jedoch mit Triethylenglykol gearbeitet, durch das eine Rohausbeute von mehr als 90 % nach dem Auskristallisieren aus einem Lösungsmittel erzielt werden kann. Mit steigendem Molgewicht des verwendeten Polyglykols nehmen jedoch die erzielbaren Rohausbeuten ab. So werden mittels eines Polyethylenglykols mit einem Molgewicht von 200 nur noch etwa 70 % Rohausbeute erhalten.

Die Rührfähigkeit der Reaktionsmasse bleibt erhalten, wenn vor dem Abdestillieren des überschüssigen Phenols mit 15 % bis 25 %, bezogen auf das Volumen der Reaktionsmasse, Glykol aufgefüllt wird.

Nach dem Abdestillieren des Phenols läßt sich das Produkt aus Dichlorethan, Toluol, einem Toluol/Isopropanolgemisch, Acetonitril und anderen mit den verwendeten Polyalkylenglykolen mischbaren Lösungsmitteln auskristallisieren. Durch eine anschließende Behandlung mit Wasser wird nach dem Trocknen 9,9-Bis-(4-hydroxyphenyl-)fluoren als weißes kristallines Produkt erhalten.

Zur Durchführung des Verfahrens werden Fluorenon und Phenol z. B. im molaren Verhältnis 1 : 6 im Reaktionsgefäß vorgelegt und β-Mercaptopropionsäure als Katalysator in einer Menge von etwa 0,01 Mol pro Mol eingesetzten Fluorenons hinzugefügt. Dieses Gemisch wird auf eine Temperatur von 50 bis 60 °C erwärmt. Unter Rühren wird trockenes Chlorwasserstoffgas in einer Menge von etwa 0,3 bis 0,45 mol pro Mol eingesetzten Fluorenons eingeleitet und noch 2 bis 6 h nachgerührt. Anschließend wird das Reaktionsgemisch mit etwa 15 Gew.-% Triethylenglykol, bezogen auf das Gesamtvolumen, verdünnt.

Anschließend wird bei vermindertem Druck und bei 40 bis 90 °C das entstandene Reaktionswasser gemeinsam mit der gelösten Salzsäure abdestilliert. Nach einem kurzen, wasserhaltigen Vorlauf kann nun das überschüssige Phenol bei 90 bis 115 °C abdestilliert werden. Der Destillationsrückstand bleibt gut rührbar und kann nun leicht in einem Lösungsmittel aufgenommen und auskristallisiert werden. Zu diesem Zweck wird der Rückstand z. B. vorsichtig unter Rückfluß mit dem 1,5-fachen Volumen an Toluol verdünnt. Die erhaltene Suspension wird unter Rühren abgekühlt. Bei etwa 80 °C erfolgt die Auskristallisation. Es wird bei etwa 5 °C abgesaugt und mit kaltem Toluol nachgewaschen. Das so abgetrennte Produkt wird nun mit Wasser aufgeschlemmt und bei 80 bis 90 °C gerührt. Nach dem Abkühlen auf etwa 20 °C wird das Fluorenderivat abgesaugt und bei etwa 110 °C im Vakuum getrocknet.

Auf diese Weise wird ein Rohprodukt mit einer Reinheit von etwa 96 % in einer theoretischen Ausbeute von mehr als 90 % erhalten. Durch Umkristallisation aus einem Gemisch aus Toluol/Isopropanol (9 : 1) kann die Reinheit auf 99,6 bis 99,9 % erhöht werden.

Vereinfacht werden kann dieses Verfahren, indem als Lösungsmittel nach dem Abdestillieren des Phenols eine entsprechende Menge Acetonitril hinzugefügt wird. Bereits nach dem Abfiltrieren des ausgefallenen kristallinen Addukts kann nach dem Trocknen im Vakuum 9,9-Bis-(4-hydroxyphenyl-)fluoren mit einer Reinheit von 98 % erhalten werden, ohne eine Wasserbehandlung durchzuführen.

Die folgenden Beispiele sollen zur näheren Erläuterung der Erfindung dienen. Diese jedoch nicht auf die Beispiele beschränken.

### Beispiele

### Beispiel 1

In einem 2 l-Dreihalskolben werden 565 g Phenol, 180 g Fluorenon und 0,8 g β-Mercaptopropionsäure unter Rühren auf 55 °C erwärmt. Innerhalb von 6 h werden langsam etwa 14 g HCl-Gas eingeleitet. Dabei wird die Temperatur beibehalten. Nachdem die Gaseinleitung beendet ist, wird noch etwa 2 h bei 55 °C nachgerührt und anschließend mit 100 ml (112 g) Triethylenglykol verdünnt.

Das entstandene Reaktionswasser wird nun gemeinsam mit der gebildeten Salzsäure unter Wasserstrahlvakuum abdestilliert. Danach wird Phenol unter vermindertem Druck abdestilliert. Den flüssigen Destillationsrückstand läßt man auf 120 °C unter Rühren abkühlen und gibt anschließend langsam unter Rückflußbedingungen und Rühren 800 ml Toluol zu, wobei sich der Destillationsrückstand löst. Wird diese Toluollösung langsam abgekühlt, erfolgt unterhalb von 80 °C eine Kristallisation. Im Eisbad wird auf eine Temperatur von 0 °C abgekühlt. Die ausgefallenen Kristalle werden abfiltriert und mit 400 ml kaltem Toluol nachgewaschen.

Anschließend wird das abgetrennte triethylenglykolhaltige Produkt in 1.000 ml Wasser aufgeschlämmt und auf 80 bis 90 °C unter Rühren erwärmt. Nachdem wieder auf etwa 20 °C abgekühlt worden ist, werden die ausgefallenen Kristalle abfiltriert und nach zweimaliger Wäsche mit je 250 ml Wasser wird das erhaltene 9,9-Bis-(4-hydroxyphenyl-)fluoren im Vakuum bei 110 °C getrocknet.
- Ausbeute:: 308 g 9,9-Bis-(4-hydroxyphenyl-)fluoren = 88 % der Theorie
- Reinheit:: 97 % (HPLC)
Durch eine Umkristallisation aus Toluol : Isopropanol 9 : 1 steigt die Reinheit auf 99,8 % (HPLC).

Abdestilliertes Phenol wird gesammelt und erneut im Verfahren eingesetzt. Das als Filtrat angefallene Toluol kann ebenfalls wieder eingesetzt werden, muß jedoch nach etwa dreimaliger Verwendung destillativ gereinigt werden.

### Beispiel 2

Die Durchführung erfolgt wie in Beispiel 1. Es wird anstelle von frischem Triethylenglykol der aus Beispiel 1 erhaltene triethylenglykolhaltige Destillationsrückstand zugegeben, der aus der Mutterlauge erhalten wird und noch nicht isoliertes Fluorenbisphenol, Nebenprodukte der Kondensationsreaktion und geringe Mengen Phenol enthält. Dieser Rückstand wird mit frischem Triethylenglykol auf 110 ml aufgefüllt und vor dem Abdestillieren des Phenols zu dem Reaktionsgemisch gegeben. Auf diese Weise wird noch im Triethylenglykol gelöstes Produkt aus der vorhergehenden Umsetzung gewonnen und nach erfolgter Aufarbeitung, wie in Beispiel 1 beschrieben, werden 322 g 9,9-Bis-(4-hydroxyphenyl-)fluoren (= 92 % der Theorie) mit einer Reinheit von 96 % (HPLC) erhalten. Durch Umkristallisation aus einem Toluol : Isopropanol-Gemisch 9 : 1 wird die Reinheit auf 99,8 % (HPLC) gesteigert.

### Beispiel 3

Die Durchführung erfolgt wie in Beispiel 1. Anstelle von Triethylenglykol gibt man 100 ml eines Polyethylenglykolgemischs mit einem durchschnittlichen Molekulargewicht von ungefähr 200 zu. Die Ausbeute des erhaltenen isolierten Produkts beträgt 245 g = 70 % der Theorie mit einer Reinheit von 98,3 % (HPLC). Durch Umkristallisation aus einem Lösungsmittelgemisch Toluol : Isopropanol (9 : 1) steigt die Reinheit des Produkts auf 99,8 % (HPLC).

### Beispiel 4

In eine Schmelze von 180 g 9-Fluorenon (1 mol), 565 g Phenol (6 mol) und 1 g Mercaptopropionsäure leitet man bei 55 °C innerhalb von 6 h 14 g Chlorwasserstoff (0,38 mol) ein und rührt noch 2 h bei 55 °C nach. Das Reaktionsgemisch wird anschließend mit 110 ml Triethylenglykol verdünnt. Das überschüssige Phenol destilliert man unter vermindertem Druck ab. Der Vorlauf enthält Salzsäure und Phenol, der Hauptlauf besteht aus 99 %-igen Phenol, der wieder in den nächsten Ansatz eingesetzt wird. Den Destillationsrückstand läßt man auf 110 °C unter Rühren abkühlen, gibt vorsichtig 500 ml Acetonitril zu und kühlt langsam bis auf 0 °C ab.

Das dabei ausfallende weiße Produkt wird abfiltriert und anschließend im Vakuum getrocknet.

Es wird eine Ausbeute an 9,9-Bis-(4-hydroxyphenyl-)fluoren von 280 g (80 % der Theorie) mit einer Reinheit von 98 % erhalten.

Durch erneute Umkristallisation aus Acetonitril wird ein 99,9 %-iges weißes kristallines Produkt erhalten.

## Patentansprüche

1. Verfahren zur Herstellung von 9,9-Bis-(4-hydroxyphenyl-)fluoren durch eine bei 30 bis 90 °C durchgeführte und durch β-Mercaptopropionsäure und HCl-Gas katalysierte Kondensationsreaktion von Fluorenon und Phenol, die in molaren Verhältnissen von 1 : 4 bis 1 : 8 vorgelegt werden, **dadurch gekennzeichnet**, daß das Reaktionsgemisch nach beendeter Reaktion mit einem Polyalkylenglykol vermischt wird, überschüssiges Phenol abdestilliert wird, der Destillationsrückstand mit einem mit Polyalkylenglykol mischbaren Lösungsmittel versetzt wird, das dabei auskristallisierende Produkt abgetrennt und mit Wasser aufgeschlämmt und erwärmt wird, wobei 9,9-Bis-(4-hydroxyphenyl-)fluoren in Form weißer Kristalle erhalten wird.

2. Verfahren gemäß Anspruch 1, **dadurch gekennzeichnet**, daß als Polyalkylenglykol ein Polyethylenglykol der allgemeinen Formel H(-O-CH₂-CH₂-)ₙ OH mit n = 2 bis 13 in das Reaktionsgemisch eingerührt wird.

3. Verfahren gemäß der Ansprüche 1 und 2, **dadurch gekennzeichnet**, daß als Polyalkylenglykol Triethylenglykol in das Reaktionsgemisch eingerührt wird.

4. Verfahren gemäß der Ansprüche 1 bis 3, **dadurch gekennzeichnet**, daß das Polyalkylenglykol in einer Menge von 15 bis 20 Vol-%, bezogen auf das Reaktionsgemisch, zugefügt wird.

5. Verfahren zur Herstellung von 9,9-Bis-(4-hydroxyphenyl-)fluoren durch eine bei 30 bis 90 °C durchgeführte und durch β-Mercaptopropionsäure und HCl-Gas katalysierte Kondensationsreaktion von Fluorenon und Phenol, die in molaren Verhältnissen von 1 : 4 bis 1 : 8 vorgelegt werden, **dadurch gekennzeichnet**, daß das Reaktionsgemisch nach beendeter Reaktion mit einem Polyalkylenglykol vermischt wird, überschüssiges Phenol abdestilliert wird, der Destillationsrückstand mit einem Acetonitril versetzt wird, das dabei auskristallisierende Addukt abfiltriert wird und durch Trocknen im Vakuum 9,9-Bis-(4-hydroxyphenyl-)fluoren erhalten wird.

## Claims

1. A process for the preparation of 9,9-bis(4-hydroxyphenyl)fluorene by means of a condensation reaction between fluorenone and phenol in molar ratios of 1:4 to 1:8 carried out at 30°C to 90°C and catalysed by β-mercaptopropionic acid and HCl gas, characterised in that the reaction mixture is mixed with a polyalkylene glycol when the reaction is complete, excess phenol is distilled off, the distillation residue is mixed with a solvent miscible with polyalkylene glycol, the product crystallising as a result is separated, suspended in water and heated, 9,9-bis(4-hydroxyphenyl)fluorene being obtained in the form of white crystals.

2. A process according to claim 1, characterised in that a polyethylene glycol of the general formula H(-O-CH₂-CH₂-)ₙOH with n = 2 to 13 is stirred into the reaction mixture as a polyalkylene glycol.

3. A process according to claims 1 and 2, characterised in that triethylene glycol is stirred into the reaction mixture as a polyalkylene glycol.

4. A process according to claims 1 to 3, characterised in that the polyalkylene glycol is added in a quantity of 15 to 20 vol.% in relation to the reaction mixture.

5. A process for the preparation of 9,9-bis(4-hydroxyphenyl)fluorene by means of a condensation reaction between fluorenone and phenol in molar ratios of 1:4 to 1:8 carried out at 30°C to 90°C and catalysed by β-mercaptopropionic acid and HCl gas, characterised in that the reaction mixture is mixed with a polyalkylene glycol when the reaction is complete, excess phenol is distilled off, the distillation residue is mixed with an acetonitrile, the adduct crystallising as a result is filtered off and 9,9-bis(4-hydroxyphenyl)fluorene is obtained by drying in a vacuum.

## Revendications

1. Procédé pour la préparation de 9,9-bis-(4-hydroxyphényl)-fluorène par une réaction de condensation de fluorénone et de phénol conduite à 30 jusqu'à 90°C et catalysée par de l'acide β-mercaptopropionique et du gaz HCl, qui sont mélangés dans des rapports molaires de 1:4 jusqu'à 1:8, caractérisé en ce que le mélange réactionnel après la fin de la réaction est mélangé avec un polyalkylèneglycol, le phénol excédentaire est séparé par distillation, au résidu de distillation on ajoute un solvant miscible avec le polyalkylèneglycol, le produit ainsi recristallisé est mis en suspension avec de l'eau et réchauffé, ce qui permet d'obtenir du 9,9-bis-(4-hydroxyphényl)-fluorène sous forme de cristaux blancs.

2. Procédé selon la revendication 1, caractérisé en ce qu'en tant que polyalkylèneglycol on introduit sous agitation un polyéthylèneglycol de la formule générale H-(OH-CH₂-CH₂)ₙ-OH avec n = 2 jusqu'à 13 dans le mélange réactionnel.

3. Procédé selon les revendications 1 et 2, caractérisé en ce qu'en tant que polyalkylèneglycol on introduit sous agitation du triéthylèneglycol dans le mélange réactionnel.

4. Procédé selon les revendications 1 à 3, caractérisé en ce que l'on ajoute le polyalkylèneglycol dans une quantité de 15 à 20% en volume, rapporté au mélange réactionnel.

5. Procédé pour la préparation de 9,9-bis-(4-hydroxyphényl)-fluorène par réaction de condensation de fluorénone et de phénol conduite à 30 jusqu'à 90°C et catalysée par de l'acide β-mercaptopropionique et du gaz HCl, fluorénone et phénol qui sont introduits dans des rapports molaires de 1:4 jusqu'à 1:8, procédé caractérisé en ce que le mélange réactionnel après la fin de la réaction est mélangé avec un polyalkylèneglycol, le phénol excédentaire est séparé par distillation, au résidu de distillation on ajoute un acétonitrile, le composé d'addition ainsi recristallisé étant séparé par filtration et par séchage sous vide, on obtient le 9,9-bis-(4-hydroxyphényl)-fluorène.
